# EUROPÄISCHE PATENTSCHRIFT

(11) **EP 0 400 592 B1**
(45) Veröffentlichungstag und Bekanntmachung des Hinweises auf die Patenterteilung: **03.08.1994**
(21) Anmeldenummer: 90110222.8
(22) Anmeldetag: 30.05.1990
(51) Int. Cl.: A61N 1/05, A61N 1/375

(54) **Adapter-Vorrichtung für Herzschrittmacher-Elektroden**
Adaptor for pacemaker electrode
Adaptateur pour électrode de stimulateur cardiaque

(30) Priorität: 02.06.1989 DE 8906745 U
(43) Veröffentlichungstag der Anmeldung: 05.12.1990
(73) Patentinhaber: VascoMed Institut für Kathetertechnologie GmbH, D-79553 Weil am Rhein (DE)
(72) Erfinder: Höcherl, Manfred, D-7888 Rheinfelden (DE); Reinhardt, Jörg, D-7889 Grenzach-Wyhlen (DE)
(74) Vertreter: Rackette, Karl, Dipl.-Phys. Dr.-Ing

(56) Entgegenhaltungen:
- DE-A- 3 304 506
- DE-U- 7 815 518
- FR-A- 2 508 716
- US-A- 3 908 668
- US-A- 4 154 248

## Beschreibung

Die Erfindung betrifft eine Adaptervorrichtung für Herzschrittmacher-Elektroden mit einem Adapterkörper aus einem isolierenden Werkstoff, der eine Klemmbüchse aus einem gut leitenden Werkstoff umschließt, die über eine durchgehende Längsbohrung, die geringfügig weiter als der Durchmesser der Schrittmacher-Elektrodenleitung der Schrittmacherelektrode ist, und eine einseitige radial verlaufende Gewindebohrung verfügt, die sich ebenfalls durch den isolierenden Adapterkörper erstreckt, und mit einer in die radial verlaufende Gewindebohrung einschraubbaren Klemmschraube, die mit einem den Gewindeschaft umschließenden und die Gewindebohrung flüssigkeitsdicht und elektrisch isolierend abdichtenden O-Ring versehen ist.

Eine solche Adaptervorrichtung ist aus der US-A-3 908 668 bekannt. Mit einer solchen Adaptervorrichtung ist es möglich, das Elektrodenkabel einer Schrittmacherelektrode sicher und fest mit dem Herzschrittmacher zu verbinden, wie dies in den Zeichnungen der US-A-3 908 668 dargestellt ist. Nun sind heutzutage, d.h. knapp 20 Jahre nach dem Anmeldetag der US-A-3 908 668, eine Vielzahl von Herzschrittmachern in Gebrauch, die über eine Steckbuchse zum einfachen Einstecken eines Elektrodenkabels verfügen. Diese Steckbuchsen sind naturgemäß zwischen einzelnen Fabrikaten von unterschiedlicher Ausgestaltung. Daher ist bei einem Ersatz des Schrittmachers bei bereits implantierter Elektrode ein einfaches Ein- und Ersetzen nicht möglich.

Vielmehr mußte bislang der operierende Arzt die Elektrode mit dem Adapter von Hand mit einem Silikonkleber verkleben. Diese während der Operation durchzuführende Verklebung ist zeitaufwendig und zeigt nicht immer die gewünschten Resultate. Das Vorgehen ist zeitaufwendig und es Dichtigkeitsmängel sind nicht immer auszuschließen. Auch mußten für eine Vielzahl von Steckertypen Adapter auf lager gehalten werden, damit der gewünschte - für den Patienten individuell ausgewählte Herzschrittmacher - an die Elektrode angeschlossen werden konnte. Dies ergab eine hohe Bevorratung der Kliniken mit Adaptern mit den unterschiedlichen Steckeranschlüssen. Dies ging zu Lasten der Übersichtlichkeit in der Lagerhaltung und auch der Kosten der Kliniken.

Eine Vereinheitlichung der Verbindungsstecker ist angesichts der Vielzahl von Herzschrittmacher-Systemen, die bei vielen Millionen Patienten implantiert sind, in absehbarer Zukunft nicht durchführbar.

Die DE-U-78 15 518 betrifft ein Werkzeug, welches bei der Verbindung eines Steckerteiles mit einer Schrittmacherelektrode einsetzbar ist. Hierbei wird das Elektrodenkabel durch das Steckerteil hindurchgeführt bzw. das Steckerteil mit Hilfe eines Schiebers auf die Elektrode aufgeschoben. Hier hat die Adaptervorrichtung nach der DE-U-78 15 518 lediglich die Funktion, eine im wesentlichen einhändige Bedienung zu gestatten, um nicht die Tülle oder das Steckerteil mit einer Hand umfassen und führen zu müssen. Sie zeigt eine Abstreifvorrichtung, ohne daß eine Möglichkeit zum seitlichen Einlegen einer Elektrode gegeben ist.

Diese Vorrichtung weist den Nachteil auf, daß keine Hilfen vorgesehen sind, um das dem Anschluß entsprechende Ende in das Steckerteil einzufädeln. Eine Verbindung einer Elektrode mit einem Herzschrittmacher oder dessen Stecker wird nicht beschrieben.

Ausgehend von diesem Stand der Technik liegt der Erfindung die Aufgabe zugrunde, eine Adaptervorrichtung zu schaffen, die es gestattet, in einfacher Weise ein beliebiges Herzschrittmacher-Elektrodenkabel mit einem Stecker in festsitzender und elektrisch gut leitender Weise zu versehen, der in einen Herzschrittmacher einsteckbar ist.

Diese Aufgabe wird erfindungsgemäß für eine Adaptervorrichtung gemäß dem Oberbegriff des Anspruchs 1 dadurch gelöst, daß auf der einen Seite des Adapterkörpers eine mit einem Schrittmacherstecker versehene Stecker-Elektrode mit der Klemmbüchse innen leitend verbunden ist und daß auf der anderen Seite des Adapterkörpers eine Abstreifvorrichtung in den aus einem elastischen Werkstoff bestehenden Adapterkörper einschiebbar ist, die einen Abstreifhalter mit einem Elektrodenführungsdorn und einen Abstreifring aufweist, der auf dem Elektrodenführungsdorn reitend verschiebbar ist, daß der hohlzylindrische Elektrodenführungsdorn über ein einseitig offenes Segment verfügt, in das die Schrittmacher-Elektrode zusammen mit ihrer isolierenden Ummantelung einlegbar ist, und daß der Abstreifring über eine seitliche Nut verfügt, deren Breite größer als die Breite des einseitig offenen Segmentes und kleiner als der Außendurchmesser des Elektrodenführungsdorn ist.

Mit Hilfe dieser Adaptervorrichtung ist eine vereinfachte Arbeitsweise für den operierenden Arzt gegeben, die zu einer sicheren dichten Verbindung zwischen der Stecker-Elektrode und der Schrittmacher-Elektrode führt. Die eigentliche Adaptervorrichtung ist fest und dicht mit der Schrittmacherelektrode und dem Schrittmacherstecker verbunden.

Der Adapterkörper aus dem elastischen Werkstoff wird nun mit seinem offenen Ende durch Aufweiten auf den Elektrodeneinführungsdorn der Abstreifvorrichtung bis zum Anschlag aufgeschoben. Nun ist es ohne jede Schwierigkeit möglich, das um ein kurzes Stück abisolierte Ende der Katheder-Elektrode durch die Elektrodeneinführungsnut des Elektrodeneinführungsdorns in die mit dieser fluchtenden Längsbohrung der Klemmbüchse des Adapterkörpers einzuführen, und zwar so weit, bis diese praktisch auf das Ende der Adapterbohrung aufstößt.

Um dieses Einführen der Katheder-Elektrode durch eine Sichtkontrolle zu überwachen, so daß auf jeden Fall eine sichere metallische und damit gut leitende Verbindung hergestellt wird ist bei einer bevorzugten Ausführungsform einer Adaptervorrichtung vorgesehen, daß eine Sichtbohrung querverlaufend zur Längsbohrung von etwa dem Durchmesser derselben zwischen der Gewindebohrung und dem Anfang einer Stecker-Elektrodenspirale der Steckerelektrode angeordnet ist. Durch diese Sichtbohrung kann das ausreichend tiefe Einführen der Katheder-Elektrode beobachtet werden, bevor durch Eindrehen der Klemmschraube bis zur Anlage des linsenförmigen Kopfes mit dem O-Ring am Adapterkörper die Elektrodenspirale in der Klemmbüchse absolut zugfest und sicher festgeklemmt ist.

Daraufhin wird mittels des Abstreifringes der Abstreifvorrichtung die letztere aus dem Adapterkörper herausgezogen, wobei sich dieser dichtend an die Isolierummantelung anlegt. Auf diese einfache Weise wird die Herstellung der zuverlässig leitenden und abgedichteten Verbindung mittels der neuen Vorrichtung ermöglicht.

Weitere Merkmale, Einzelheiten und Vorteile der Erfindung sind den in den Zeichnungen schematisch dargestellten Ausführungsbeispielen zu entnehmen, welche nachstehend näher erläutert sind.

Es zeigen
- Fig. 1: eine Adaptervorrichtung in Seitenschnitt mit Schraubendreher;
- Fig. 2: einen Adapter in Seitenschnitt;
- Fig. 3 und 5: einen Führungsdorn mit Haltering in Seitenanansicht;
- Fig. 4: einen Führungsdorn mit Haltering in Draufsicht;
- Fig. 6 und 7: einen Abstreifring in Seitenan- und Draufsicht;
- Fig. 8: einen Führungsdorn mit Halte- und Abstreifring, das Aufweit- und Einführwerkzeug, in Seitenansicht.

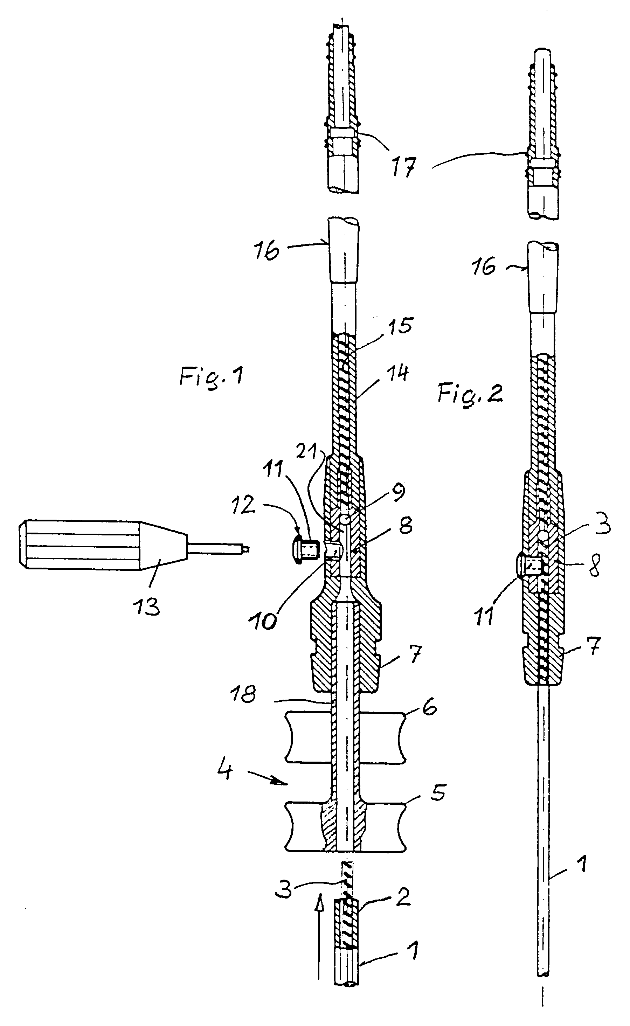

Die Fig. 1 zeigt eine Adaptervorrichtung nach der Lehre der Erfindung, die zur Verbindung implantierter Herzschrittmacherelektroden dient. Die Stecker-Elektrode 16 mit dem Stecker 17 für den nicht dargestellten Herzschrittmacher besteht aus der fest mit der metallischen, leitenden Klemmbüchse 8 verbunden Stecker-Eleketrodenspirale 15 und der Silikonummantelung 14. Die Stecker-Silikonummantelung 14 wird flüsgigkeitsdicht und elektrisch isolierend von dem Silikonkörper 7 umgeben. Die Klemmbüchse 8 ist mit einer Längsbohrung 21 zum Einstecken der abisolierten Schrittmacher-Elektrodenspirale 3 der Schrittmacher-Elektrode 1 und an ihrer zylindrischen Außenfläche mit einer in radialer Richtung verlaufenden Gewindebohrung 10 für die Klemmschraube 11 und mit einem querverlaufend durchgehenden Sichtloch 9 versehen. Die Klemmschraube 11 wird mit dem Imbusschraubendreher 13 festgezogen. Die Klemmbüchse 8 ist von dem Silikonkörper 7 umschlossen, der auf den halbrunden, offenen Führungsdorn 18 aufgestülpt ist. In den Führungsdorn 18 kann die Schrittmacher-Elektrode 1 mit der Silikonummantelung eingelegt werden.

Die Fig. 2 zeigt den Adapter als Verbindung zwischen der Stecker-Elektrode 16 und der Schrittmacher-Elektrode1. In die mit der Stecker-Elektrodenspirale 15 verbundene Klemmbüchse 8 ist die Schrittmacher-Elektrode 1 mit der Schrittmacher-Elektrodenspirale 3 ohne die Schrittmacher-Silikonummantelung 2 eingesteckt. Die Klemmschraube 11 mit dem O-Ring 12 preßt auf die Elektrodenspirale 3 und sichert diese gegen Herausrutschen aus der Klemmbüchse 8. Der Silikonkörper 7 liegt mit Spannung flüssigkeitsdicht und elektrisch isolierend über der Silikonummantelung 2 der Schrittmacher-Elektrode 1.

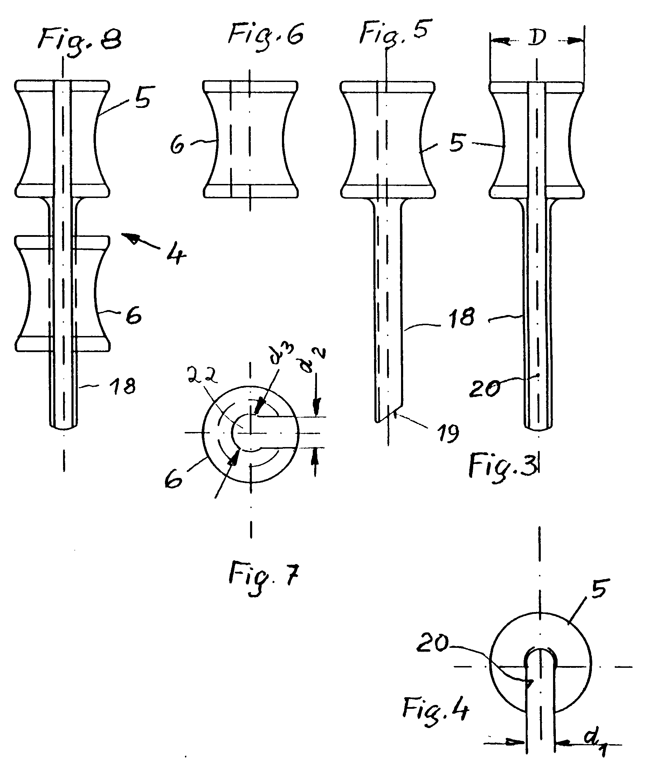

Die Fig. 3 und 5 zeigen die Seitenansicht eines aus einem Teil bestehenden Führungsdornes 18 und Halteringes 5. In Fig. 4 ist der Führungsdorn 18 und Haltering 5 in Draufsicht dargestellt. Die Schrittmacher-Elektrode 1 kann in das Segment 20 des Führungsdorns 18 eingelegt werden. Die Breite d₁ des Segmentes 20 ist größer als der Durchmesser der Silikonummantelung 2 der Schrittmacher-Elektrode 1, welche koaxial zu dem Führungsdorn 18 verschiebbar und in das Segment 20 des Halteringes 5 einlegbar ist.

In Fig. 6 ist ein Abstreifring 6 in Seitenansicht, in Fig. 7 in Draufsicht dargestellt. Die Führungsbohrung 22 weist einen größeren Durchmesser d₃ auf, als der Führungsdorn 18, um auf diesem koaxial verschiebbar zu sein. Die Breite d₂ des Spaltes ist zum Einlegen der Schrittmacher-Elektrode 1 breiter als deren Durchmesser. Die Breite d₂ ist kleiner als der Durchmesser des Führungsdornes 18.

In Fig. 8 ist eine Abstreifvorrichtung 4, bestehend aus dem Haltering 5 und dem koaxial des Führungsdornes 18 verschiebbaren Abstreifring 6, dargestellt. Da der Spalt am Abstreifring 6 nach außen enger als der größte Durchmesser des Führungsdornes 18 wird, kann der Abstreifring 6 nicht radial vom Führungsdorn 18 abgenommen werden, sondern muß koaxial aufgesteckt werden.

Die Konnektion einer implantierten Herzschrittmacherelektrode an einen Herzschrittmacher mit der Adapter-Vorrichtung ist anhand der Fig. 1 im folgendem beschrieben.

Die Schrittmacher-Elektrode 1 wird in das Segment 20 des Führungsdornes 18 und des Halterings 5 eingelegt, wobei der Abstreifring 6 mit dem Spalt zum Durchführen zur Schrittmacher-Elektrode 1 hin gedreht ist. Dann wird die Schrittmacher-Elektrode 1 koaxial zum Führungsdorn 18 verschoben, bis die auf ca. 7 mm von der Silikonummantelung 2 abisolierte Elektrodenspirale 3 in der Sichtbohrung 9 erscheint. Nach Eindrehen der Klemmschraube 11 zum Festhalten der Elektrodenspirale 3 in der Klemmbüchse 8 mit dem Schraubendreher 13 wird der Abstreifring 6 längs des Führungsdorns 18 von dem Haltering 5 weg verschoben. Der Silikonkörper 7 wird vom Führungsdorn 18 abgestreift und umschließt eng anliegend die Schrittmacher-Silikonummantelung 2 flüssigkeitsdicht und elektrisch isolierend. Die Abstreifvorrichtung 4 kann für eine weitere Adapter-Vorrichtungen wiederverwendet werden.

## Patentansprüche

1. Adaptervorrichtung für Herzschrittmacher-Elektroden mit einem Adapterkörper (7) aus einem isolierenden Werkstoff, der eine Klemmbüchse (8) aus einem gut leitenden Werkstoff umschließt, die über eine durchgehende Längsbohrung (21), die geringfügig weiter als der Durchmesser der Schrittmacher-Elektrodenleitung (3) der Schrittmacherelektrode (1) ist, und eine einseitige radial verlaufende Gewindebohrung (10) verfügt, die sich ebenfalls durch den isolierenden Adapterkörper (7) erstreckt, und mit einer in die radial verlaufende Gewindebohrung (10) einschraubbaren Klemmschraube (11), die mit einem den Gewindeschaft umschließenden und die Gewindebohrung (10) flüssigkeitsdicht und elektrisch isolierend abdichtenden O-Ring (12) versehen ist, **dadurch gekennzeichnet,**
daß auf der einen Seite des Adapterkörpers (7) eine mit einem Schrittmacherstecker (17) versehene Stecker-Elektrode (16) mit der Klemmbüchse (8) innen leitend verbunden ist und daß auf der anderen Seite des Adapterkörpers (7) eine Abstreifvorrichtung (4) in den aus einem elastischen Werkstoff bestehenden Adapterkörper (7) einschiebbar ist, die einen Abstreifhalter (5) mit einem Elektrodenführungsdorn (18) und einen Abstreifring (6) aufweist, der auf dem Elektrodenführungsdorn (18) reitend verschiebbar ist, daß der hohlzylindrische Elektrodenführungsdorn (18) über ein einseitig offenes Segment (20, d1) verfügt, in das die Schrittmacher-Elektrode (1) zusammen mit ihrer isolierenden Ummantelung (2) einlegbar ist, und daS der Abstreifring (6) über eine seitliche Nut verfügt, deren Breite (d2) größer als die Breite (d1) des einseitig offenen Segmentes (20) und kleiner als der Außendurchmesser (d3) des Elektrodenführungsdorn (18) ist.

2. Adaptervorrichtung nach Anspruch 1, dadurch gekennzeichnet, daß eine Sichtbohrung (9) querverlaufend zur Längsbohrung (21) von etwa dem Durchmesser derselben zwischen der Gewindebohrung (10) und dem Anfang einer Stecker-Elektrodenspirale (15) der Steckerelektrode (16) angeordnet ist.

3. Adaptervorrichtung nach Anspruch 1, dadurch gekennzeichnet, daß eine Sichtbohrung (9) querverlaufend zur Längsbohrung (21) von etwa dem Durchmesser derselben zwischen der Gewindebohrung (10) und dem Ende der Längsbohrung (21) angeordnet ist.

## Claims

1. An adapter device for pacemaker electrodes having an adapter body (7) comprising an insulating material which encloses a clamping sleeve (8) of a material which is a good conductor, which sleeve has a longitudinal bore (21) which extends therethrough and which is slightly wider than the diameter of the pacemaker electrode line (3) of the pacemaker electrode (1), and a radially extending screwthreaded bore (10) at one side, which also extends through the insulating adapter body (7), and further having a clamping screw (11) which can be screwed into the radially extending screwthreaded bore (10) and which is provided with an O-ring (12) which surrounds the screwthreaded shank and which fluid-tightly and electrically insulatingly seals off the screwthreaded bore (10), characterised in that on the one side of the adapter body (7) a plug electrode (16) which is provided with a pacemaker plug (17) is internally conductively connected to the clamping sleeve (8) and that on the other side of the adapter body (7) a stripper device (4) can be pushed into the adapter body (7) which comprises an elastic material, the stripper device (4) having a stripper holder (5) with an electrode guide bar (18) and a stripper ring (6) which is displaceable slidably on the electrode guide bar (18), that the hollow-cylindrical electrode guide bar (18) has a segment (20, d1) which is open at one side and into which the pacemaker electrode (1) can be inserted together with its insulating sheathing (2), and that the stripper ring (6) has a lateral groove, the width (d2) of which is larger than the width (d1) of the segment (20) which is open at one side, and smaller than the outside diameter (d3) of the electrode guide bar (18).

2. An adapter device according to claim 1 characterised in that a viewing bore (9) is arranged extending transversely with respect to the longitudinal bore (21) of approximately the diameter thereof between the screwthreaded bore (10) and the beginning of a plug electrode coil (15) of the plug electrode (16).

3. An adapter device according to claim 1 characterised in that a viewing bore (9) is arranged extending transversely with respect to the longitudinal bore (21) of approximately the diameter thereof between the screwthreaded bore (10) and the end of the longitudinal bore (21).

## Revendications

1. Adaptateur pour électrode de stimulateur cardiaque avec un corps d'adaptateur (7) en matériau isolant, entourant une douille de serrage (8) en matériau bon conducteur, laquelle est traversée par un conduit longitudinal de diamètre faiblement supérieur à celui du conducteur de l'électrode (3) du stimulateur et comporte un taraudage radial (10) latéral qui traverse également le corps isolant (7) de l'adaptateur, l'adaptateur étant équipé d'une vis de serrage (11) coopérant avec le taraudage (10) radial, ladite vis étant pourvue d'un joint torique (12) entourant l'axe fileté pour empêcher les fuites au niveau du taraudage et pour réaliser l'isolation électrique, caractérisé en ce que sur un côté du corps (7) de l'adaptateur, une électrode de connexion (16) pourvue d'une fiche mâle de stimulateur (17) est reliée intérieurement à la douille (8) conductrice, et caractérisé en ce que sur son autre côté l'adaptateur présente un dispositif d'enlèvement (4) dans lequel le corps d'adaptateur (7) en matériau élastique est enfichable, l'adaptateur étant composé d'un support d'enlèvement (5), d'un mandrin d'électrode (18) et d'un anneau de retrait (6) pouvant coulisser de façon chevauchante sur le mandrin d'électrode (18), et caractérisé en ce que le mandrin d'électrode (18) cylindrique creux possède sur une de ses extrémités un segment (20, d1) ouvert, dans lequel on introduit l'électrode de stimulation (1) avec son entourage isolant, et en ce que l'anneau de retrait (6) possède une rainure latérale dont la largeur (d2) est supérieure à la largeur interne (d1) du segment ouvert (20) et inférieure au diamètre externe (d3) du mandrin d'électrode (18).

2. Adaptateur selon la revendication 1, caractérisé en ce qu'un trou de visualisation (9) disposé transversalement à l'alésage longitudinal (21) et de diamètre à peu près égal, est disposé entre le taraudage radial (10) et le début du connecteur d'une électrode en spirale (15) de l'électrode de connexion (16).

3. Adaptateur selon la revendication 1, caractérisé en ce que le trou de visualisation (9) disposé transversalement à l'alésage longitudinal (21) et de diamètre à peu près égal, est disposé entre le taraudage radial (10) et la fin de l'alésage longitudinal (21).
